# EUROPÄISCHE PATENTSCHRIFT

(11) **EP 3 103 506 B1**
(45) Veröffentlichungstag und Bekanntmachung des Hinweises auf die Patenterteilung: **15.05.2024**
(21) Anmeldenummer: 16172381.2
(22) Anmeldetag: 01.06.2016
(51) Int. Cl.: A61M 39/10, F16L 41/00, A61M 39/12, F16L 33/00, F16L 33/18, A61M 1/14

(54) **KUPPLUNG FÜR SCHLAUCHVERBINDUNGEN**
COUPLING FOR HOSE CONNECTIONS
COUPLAGE POUR DES RACCORDS DE CONDUITE

(30) Priorität: 10.06.2015 DE 102015109158
(43) Veröffentlichungstag der Anmeldung: 14.12.2016
(73) Patentinhaber: B. Braun Avitum AG, 34212 Melsungen (DE)
(72) Erfinder: Lisitschew, Swetlana, 80639 München (DE)
(74) Vertreter: Winter, Brandl - Partnerschaft mbB

(56) Entgegenhaltungen:
- EP-A2- 0 821 192
- WO-A2-2004/073764
- WO-A2-2014/201358
- DE-A1-102005 002 552
- DE-U1-202006 005 685
- DE-U1-202009 011 641
- FR-A1- 2 939 862
- US-A1- 2015 144 717

## Beschreibung

Die vorliegende Erfindung betrifft eine Blutbehandlungsmaschine mit einer Schlauchhalterung gemäß dem Oberbegriff des Patentanspruchs 1.

### Hintergrund der vorliegenden Erfindung

Insbesondere bei Blutbehandlungsmaschinen wie Dialysemaschinen sind einzelne Maschinenkomponenten, beispielsweise Wärmetauscher, Pumpen, Dialysator, etc. miteinander fluidtechnisch durch biegeflexible Schläuche gekoppelt. Durch die Verwendung von derartigen Schläuchen ist es möglich, die betreffenden Maschinenkomponenten einzeln oder in gewählter Zuordnung in/auf Auszügen zu montieren, die dann zu Reparatur- und/oder Wartungszwecken aus dem Maschinengehäuse teilweise gezogen werden können, um deren Zugänglichkeit zu verbessern.

Um Schlauchchaos zu vermeiden und das Risiko von Beschädigungen einzelner Schläuche zu reduzieren, macht man sich die insbesondere aus der Fahrzeugtechnik bekannte Maßnahme des Zusammenfassens von Leitungen zu sogenannten Leitungsbäumen zunutze. In anderen Worten ausgedrückt werden einzelne ausgewählte Schläuche im einfachsten Fall durch Kabelbinder über eine vorbestimmte Schlauchstrecke mechanisch zusammengefasst. Dadurch erhöht sich deren Gesamt-Knickstabilität, sodass das Beschädigungsrisiko verringert werden kann. Gleichzeitig reduziert sich das Schlauchwirrwarr zu einigen wenigen "Schlauchbäumen", die übersichtlicher angeordnet werden können.

Zur Halterung der Schläuche an/in der Maschine dienen vornehmlich die Maschinenkomponenten selbst, welche miteinander verschlaucht sind. Im Übrigen werden die Schläuche partiell einzeln und partiell gebündelt frei im Raum verlegt, wobei weder Schlauchführungen noch Schlauchbefestigungen vorgesehen sind, welche zur Positionierung/Festlegung der Schläuche in der Maschine beitragen.

### Stand der Technik

Aus der EP 2 372 330 ist ein Luftblasendetektor als eine beispielhafte Komponente einer Blutbehandlungsmaschine bekannt. Dieser Detektor weist eine am Maschinengehäuse fixierbare Halterung auf, an der zumindest ein Ultraschallsensor angeordnet ist, um Luft und/oder Gasblasen in einer strömenden Flüssigkeit zu erfassen. In der Halterung ist hierfür ein Strömungskanal integriert, der Anschlussstücke oder Stutzen aufweist, an die Verbindungsschläuche anschließbar sind. Auf diese Weise wird nicht nur der Detektor sondern auch die daran angeschlossenen Schläuche am Gehäuse gehalten.

Ferner ist aus der EP 2 672 611 eine Schlauchanschlusseinrichtung (fluidtechnische Steckdose) bekannt, die in einer Öffnung eines Gehäuses eines medizinischen Geräts lösbar anbringbar ist und ein Verbindungselement aufweist, an welches ein Einmalartikel außerhalb des Gehäuses wahlweise anschließbar ist. D.h. es ist ein fluidtechnischer Anschluss (Steckdose) für ein externes Schlauchsystem offenbart, der in einer entsprechend dimensionierten Durchgangsbohrung eines Maschinengehäuses fixierbar/fixiert ist. Das Gehäuse-außenseitige Verbindungselement des bekannten Anschlusses, beispielsweise ein Luer-Anschluss, ist über einen hohlen Steg als Mittenabschnitt der Schlauchanschlusseinrichtung mit einem Anschlussstutzen für das Gehäuse-innere Schlauchsystem verbunden, der durch die Öffnung des Gehäuses in das Gehäuseinnere einführbar ist. Darüber hinaus hat die Schlauchanschlusseinrichtung ein separates Federelement, das lösbar so am Steg anbringbar ist (vergleichbar eines Axialsicherungsrings), dass die Schlauchanschlusseinrichtung über das Federelement am Gehäuse axial federnd abgestützt ist. Insofern stellt auch diese bekannte Schlauchanschlusseinrichtung ein weiteres Beispiel einer Maschinenkomponente im vorstehend beschriebenen Sinne, vorliegend für das fluidtechnische Anschließen von externen Einrichtungen, dar.

Weitere medizinische Geräte sind aus den Dokumenten WO 2004/073964 A1 sowie WO 2014/201358 A1 bekannt. Die WO 2004/073964 A1 offenbart beispielsweise eine Blutbehandlungsmaschine gemäß dem Oberbegriff des Anspruchs 1.

Die Dokumente US 2015 / 144717 A1, DE 20 2009 011641 U1, DE 10 2005 002552 A1, DE 20 2006 005 685 U1, EP 0 821 192 A2, sowie FR 2 939 862 A1 widmen sich der Kopplung zwischen einem Schlauch und einem weiteren Bauteil und betreffen keine Blutbehandlungsmaschinen.

Aus der vorstehenden Beschreibung zum Stand der Technik ist ersichtlich, dass das Halten und Fixieren von Schläuchen innerhalb einer Blutbehandlungsmaschine zumeist nur per vorhandener Maschinenkomponenten erfolgt und darüber hinaus die Schläuche lose gehalten sind. Dies hat zur Folge, dass für den Fall, dass bei einer Wartung oder Reparatur einzelne Schubfächer/Auszüge, auf denen Maschinenkomponenten gelagert sind, aus dem Maschinengehäuse teilweise herausgezogen und anschließend wieder zurückgeschoben werden, die daran angeordneten Schläuche geknickt, geklemmt oder sogar abgerissen werden können. Diese Form der Beschädigung lässt sich auch durch das Zusammenfassen der Schläuche zu Schlauchbäumen oder - bündeln nicht unbedingt beseitigen.

Es besteht natürlich grundsätzlich die theoretische Möglichkeit, die miteinander verschlauchten Maschinenkomponenten so im Maschinengehäuse zu platzieren, dass die Schläuche möglichst raumstabil gehalten werden. Dieser Maßnahme sind in der Praxis jedoch Grenzen gesetzt, da die einzelnen Maschinenkomponenten unterschiedliche Funktionen und damit auch Baugrößen haben, auf die bei deren Anordnung im Maschinengehäuse Rücksicht genommen werden muss. Außerdem sind funktionsbedingt einzelne Komponenten an unterschiedlichen vorbestimmten Stellen im Maschinengehäuse zu platzieren, und daher in ihrer Anordnung nicht oder wenig variabel.

### Kurzbeschreibung der Erfindung

In Anbetracht dieser Problematik ist es die Aufgabe der vorliegenden Erfindung, eine Schlauchhaltemöglichkeit für Schläuche einer Blutbehandlungsmaschine bereitzustellen, die möglichst einfach und ohne größeren zusätzlichen Aufwand anwendbar ist und die in Ergänzung zu den ohnehin vorhandenen Maschinenkomponenten der Blutbehandlungsmaschine gemäß vorstehender Definition die einzelnen Schläuche raumstabiler halten können.

Diese Aufgabe wird durch eine Blutbehandlungsmaschine mit einer Schlauchhalterung gemäß den Merkmalen des Anspruchs 1 gelöst.

Ein Grundgedanke der vorliegenden Erfindung besteht darin, geeignete Elemente der Verschlauchung (ungleich der Maschinenkomponenten gemäß vorstehender Definition) zusätzlich zu Schlauchhalterungen umzufunktionieren. Diese geeigneten Elemente sollten vorzugsweise Bestandteil der Verschlauchung sein und eine ausreichende Steifigkeit/Stabilität haben, um Haltekräfte in ein Maschinengehäuse übertragen zu können und so die Schläuche in Raumlage beschädigungsfrei zu fixieren.

Hierbei sei darauf hingewiesen, dass sich einfache Kabelbinder, welche bekannter Maßen um eine flexible Leitung wie Kabel geführt werden können, wenig für flexible Fluidschläuche eignen, da sie die Schläuche linienartig eindrücken würden und damit Sollknickstellen bilden können. Ähnlich verhalten sich sogenannte Rohrschellen, die zwar eine flächigere Auflage bieten, aber immer noch das Problem eines Zusammendrückens des flexiblen Schlauchs haben und daher nur für starre Rohrleitungen geeignet sind.

Als besonders geeignete Elemente dieser Gattung haben sich daher sogenannte Schlauchverbinder und/oder Weichen erwiesen. Hierbei handelt es sich um vergleichsweise starre Verschlauchungselemente vorzugsweise aus Kunststoff oder einem Metall, mittels denen zwei Schläuche fluidtechnisch fest miteinander verbunden werden/sind und/oder ein Schlauch in zwei oder mehrere weitere Schläuche verzweigt wird/ist.

Im einfachsten Fall ist ein solches Schlauchverbindungselement eine starre Hülse vorbestimmter Länge, auf die von beiden Seiten jeweils ein Schlauch radial außenseitig aufgezogen werden kann. Es ist aber auch möglich, dass das Schlauchverbindungselement gleichen Funktionsprinzips ein T-, Y- oder X-Stück ist, das in diesem Fall zusätzlich als Strömungsweiche der Verschlauchung dient. Derartige Schlauchverbindungselemente sind Bestandteil der Verschlauchung selbst und nicht dafür vorgesehen, im Regelbetrieb der Blutbehandlungsmaschine wahlweise gekoppelt oder entkoppelt zu werden. Vielmehr sind es solche Elemente, die für die Bereitstellung der Verschlauchung (des Fluidstromnetzes) dienen und daher als "nicht wahlweise lösbar" beabsichtigt/vorgesehen sind.

Erfindungsgemäß ist es somit vorgesehen, ein solches (fixes) Schlauchverbindungselement zum (festen, d. h. nicht zum wahlweisen Lösen und Kuppeln angepassten) Verbinden von Schläuchen mit wenigstens einem Stromkanal oder Hülsenabschnitt ferner mit einem flexiblen Kragen (integral) auszubilden oder zu versehen, der axial von einem radial äußeren, vorzugsweise starren Anschlagsvorsprung am Stromkanal oder dem zumindest einen Hülsenabschnitt beabstandet ist, um zwischen sich und dem Anschlagsvorsprung einen Axialspalt (bzw. Außen-Umfangsnut) zu definieren.

Ein solches Schlauchverbindungselement kann dann nahezu an beliebiger Stelle im Maschinengehäuse mit einer Gehäusewand oder einem am Maschinengehäuse fixierbaren Haltebügel zusammenwirken, indem die Gehäusewand oder der Haltebügel eine Durchgangsbohrung in etwa vom Durchmesser des zumindest einen Hülsenabschnitts (bzw. kleiner als der Außendurchmesser des flexiblen Kragens) aufweist, in welches dann das Schlauchverbindungselement an seinem wenigstens einen Hülsenabschnitt unter flexibler temporärer Verformung des Kragens einsteckbar ist. Dadurch wird die Wand oder der Haltebügel in der Umfangsnut zwischen Kragen und Anschlag axial gehalten, sodass nunmehr beidseits des zumindest einen Hülsenabschnitts jeweils ein Schlauch aufgezogen werden kann.

Die Halterung ist also erfindungsgemäß das Verschlauchungs-interne Schlauchverbindungselement (und nicht eine Maschinenkomponente), das ohne den Zusatz eines daran integral angeordneten Haltearms oder dergleichen Haltevorrichtung mit der Gehäusewand und/oder am Gehäuse montierten Haltebügel für eine Krafteinleitung zusammenwirkt. Der flexible Kragen ermöglicht dabei die einseitige, einfache Montage ohne zusätzliche Arretiermittel.

Der Kragen hat eine Dübelform, derart, dass der damit versehene zumindest eine Hülsenabschnitt des Schlauchverbindungselements einfach von einer Seite in das Aufnahmeloch der Gehäusewand oder des daran montierten Haltebügels einsteckbar ist, und der Kragen dann auf der anderen Seite selbsttätig/federelastisch wieder aufspreizt und so die Wand/den Bügel zwischen sich und dem hülsenseitigen Anschlag axial hält.

Vorzugsweise kann das Schlauchverbindungselement eine einfache Schlauchhülse sein, auf deren beiden Hülsenenden ein Schlauch aufgezogen ist oder es ist eine Stromweiche beispielsweise in Form einer Y-, T-, X- oder dergleichen Verzweigungsstücks, bei welchem eines der mehreren Hülsenabschnitte zum Aufziehen von Schlauchenden mit dem vorstehend beschriebenen Kragen und vorzugsweise dem zusätzlichen Axialanschlag versehen bzw. ausgebildet ist.

Durch die Erfindung können die vorliegenden Vorteile erzielt werden:
- Schläuche werden definiert im Raum der Blutbehandlungsmaschine verlegt. Dadurch können Beschädigungen, Abknicken oder Klemmen von Schläuchen vermieden werden.
- Schläuche können in der Blutbehandlungsmaschine (Dialysemaschine) mit Einschubtechnik direkt durch die Einschubbleche geführt werden, die dann als verschiebbare Gehäusewände gelten. Dadurch müssen keine Umwege "außerhalb" vom Einschubblech genommen werden. Dabei werden sie definiert im Maschinenraum fixiert und gleichzeitig zugentlastet. Ferner können Schlauchlängen und somit das Schlauchvolumen reduziert werden. Dies hat einen positiven Einfluss beispielsweise auf den Dialysierflüssigkeitsverbrauch, den Energieverbrauch, den Desinfektionsmittelverbrauch, die Desinfektionszeit, etc..
- Schläuche können durch fixe Gehäusewände geführt werden. Dabei werden sie definiert fixiert, zugentlastet und gleichzeitig in der Gehäusedurchführung gedichtet. Dadurch kann ein unbeabsichtigtes Abreißen von Schläuchen an Verbindungsstellen mit Maschinenkomponenten vermieden werden.
- Es ist eine einseitige (Einhand-) Montage des Schlauchverbindungselements möglich durch einfaches Einstecken oder Einschieben des zumindest einen Hülsenabschnitts in das Durchgangsloch an der Maschinenwand oder dem Haltebügel. Hierbei ist kein zusätzliches axiales Fixieren erforderlich.
- Die Funktionen "Kupplung", "Durchführung", "Zugentlastung", "Schlauchverbindung" und "Schlauchfixierung" werden in einem Teil zusammengefasst.

### Figurenbeschreibung

Die Erfindung wird nachstehend anhand bevorzugter Ausführungsbeispiele unter Bezugnahme auf die begleitenden Figuren näher erläutert.
Fig. 1 zeigt das Funktionsprinzip eines erfindungsgemäßen Schlauchverbindungselements anhand einer beispielhaften Y-Weiche,
Fig. 2 zeigt die Seiten- sowie die zugehörige Schnittansicht des erfindungsgemäßen Schlauchverbindungselements anhand der beispielhaften Y- Weiche in vergrößerter Darstellung,
Fig. 3 zeigt die Seiten- sowie die zugehörige Schnittansicht des erfindungsgemäßen Schlauchverbindungselements anhand der beispielhaften Y- Weiche in eingebautem Zustand und
Fig. 4a-e zeigen Varianten für ein Schlauchverbindungselement gemäß der vorliegenden Erfindung.

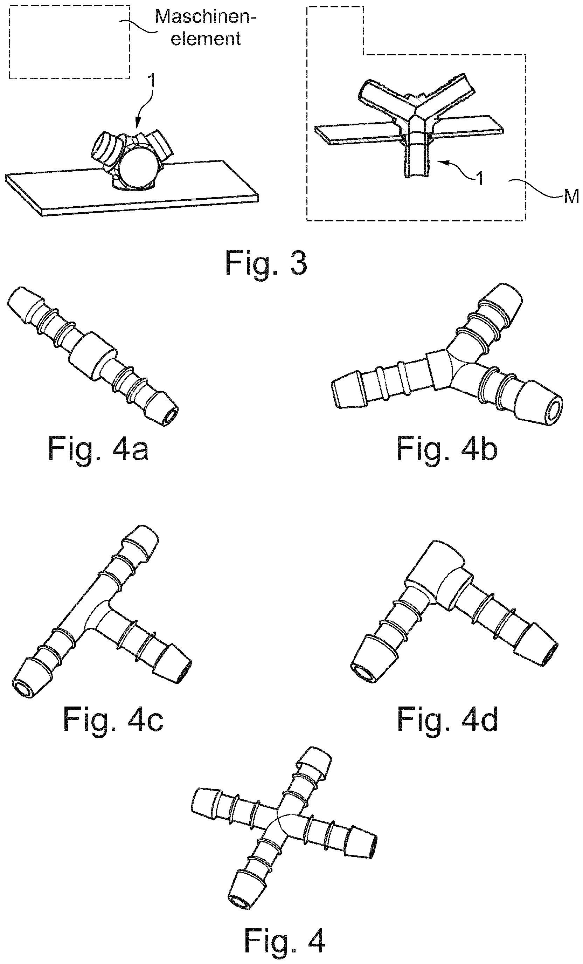

Wie aus den Figuren grundsätzlich zu entnehmen ist, ist das erfindungsgemäße Schlauchverbindungselement 1 vom Prinzip aufgebaut wie bekannte Schlauchverbinder, sodass an dieser Stelle auf den allgemeinen Stand der Technik verwiesen werden kann. So kann das erfindungsgemäße Schlauchverbindungselement (Schlauchverbinder) 1 gemäß der Fig. 4a-e die Form eines hülsenartigen Doppelstutzens, Y-Weiche, T-Weiche, Winkelstücks oder X-, bzw. Kreuzweiche aufzeigen, wobei die einzelnen Hülsenabschnitte vorzugsweise aus einem Kunststoff oder Metall zu einem einzigen starren Bauteil beispielsweise durch Spritzgießen, Verschrauben oder Stecken integral zusammengefasst sind. Diese (an sich bekannten) Formen sind auch alle als Reduzier-Schlauchverbinder denkbar, etwa wenn von einem kleindurchmessrigen Schlauch auf einen großdurchmessrigen Schlauch oder umgekehrt gewechselt werden soll. Diese Geometrien übernehmen daher grundsätzlich die Funktion einer Verschlauchungs-internen (im Regelbetrieb nicht für das wahlweise Lösen vorgesehenen) Schlauchverbindung.

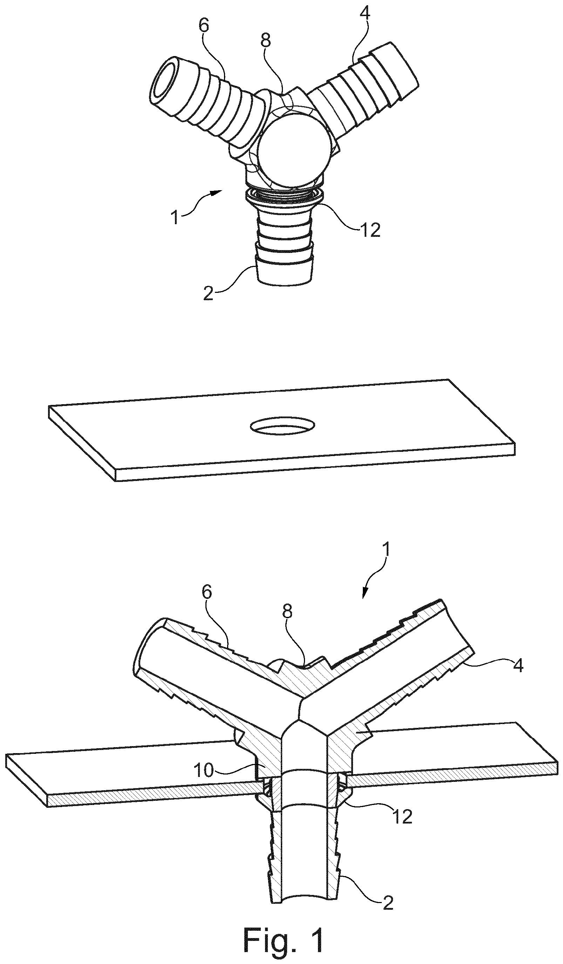

In den Fig. 1 bis 3 ist nunmehr die erfindungsgemäße Modifikation der vorstehend beschriebenen Grundformen beispielhaft anhand einer Y-Weiche gezeigt, wobei an dieser Stelle ausdrücklich darauf hingewiesen wird, dass die nachfolgend beschriebene Modifikation für alle gezeigten (und darüber hinaus noch bekannten) Formen für Schlauchverbinder der vorliegenden Gattung vorsehbar ist.

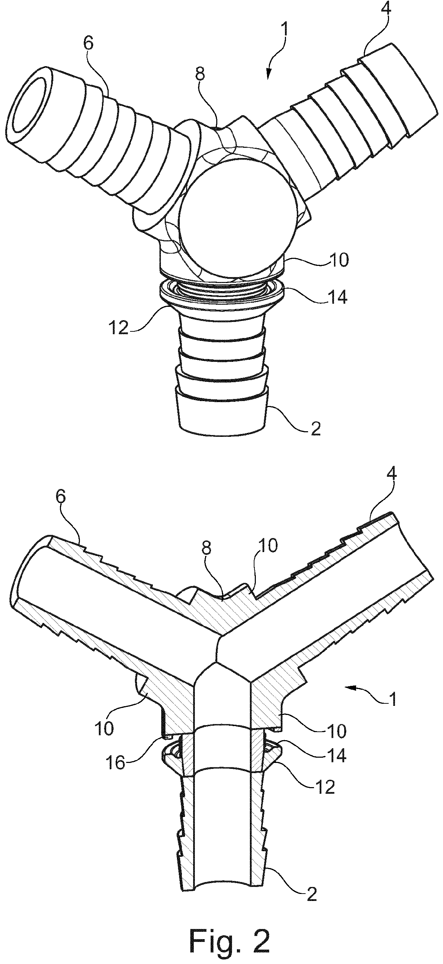

Gemäß der Fig. 2 hat die beispielhaft gezeigte Y-Weiche als das erfindungsgemäße Schlauchverbindungselement/Schlauchverbinder 1 insgesamt drei Hülsenabschnitts 2, 4, 6 für das darauf Aufziehen von miteinander zu verbindenden Schlauchenden (siehe insbesondere Fig. 3), die sämtlich stoßweise in einem zentralen Weichenknoten 8 zusammengeführt sind, derart, dass die Hülsenabschnitte 2, 4, 6 miteinander fluidgekoppelt werden. Der zentrale Weichenknoten 8 stellt dabei eine Materialanhäufung dar, wodurch sich an wenigstens einem Hülsenabschnitt 2 (vorzugsweise an allen Hülsenabschnitten (2-6) ein außenseitiger Axialanschlag 10 bildet, der normaler Weise als axialer Aufziehanschlag für das jeweils darauf aufgezogene Schlauchende dient. Da der zentrale Weichenknoten 8 und vorzugsweise auch die Hülsenabschnitte 2-6 aus einem starren Material wie Kunststoff (Thermoplast) oder Metall bestehen, ist auch der zumindest eine Axialanschlag 10 starr und im Wesentlichen formstabil.

Zusätzlich zu dem Axialanschlag 10 hat der wenigstens eine Hülsenabschnitt 2 einen vom Axialanschlag 10 axialbeabstandeten Kragen oder Tülle 12 aus einem gegenüber dem Axialanschlag 10 flexiblen (elastischen) Material wie beispielsweise einem Elastomere, wodurch sich zwischen dem Axialanschlag 10 und dem Kragen 12 eine radial äußere Umfangsnut ausbildet.

Anhand der Fig. 2 ist zu ersehen, dass der Kragen 12 in seinem Längsschnitt eine Kegel- oder Dübelform hat, derart, dass es sich ausgehend von seinem dem Axialanschlag 10 zugewandten Ende in Richtung äußerem/freien Ende des Hülsenabschnitts 2 radial (napfartig) verjüngt. Darüber hinaus bildet der Kragen 12 an seiner dem Axialanschlag 10 zugewandten Stirnseite eine axial vorragende, sowie geschlossen umlaufende Dichtlippe 14 aus, die einer an der Stirnseite des Axialanschlags 10 entsprechend ausgebildeten/angeordneten, geschlossen umlaufenden Dichtlippe 16 gegenüberliegt.

Der Kragen 12 kann auf den einen Hülsenabschnitt 2 aufgeklebt, aufgespannt der mit diesem einstückig ausgebildet sind. In jedem Fall bildet er nach seiner Ausbildung/Montage vorzugsweise eine Einheit mit dem einen Hülsenabschnitt 2 und damit Bestandteil des erfindungsgemäßen Schlauchverbindungselements 1.

Die Umfangsnut hat eine Nutbreite, die das darin Aufnehmen der Gehäusewand/daran montierten Haltebügels einer Blutbehandlungsmaschine oder Dialysemaschine M erlaubt, die in Fig. 3 nur als Systemgrenze dargestellt ist

In anderen Worten ausgedrückt, ist der Kragen 12 so an dem wenigstens einen Hülsenabschnitt 2 platziert, dass der zwischen sich und dem Axialanschlag 10 ausgebildete Axialabstand im Wesentlichen der Breite einer Gehäusewand oder eines daran montierten Haltebügels der Blutbehandlungsmaschine entspricht. Eine solche Gehäusewand oder Haltebügel ist symbolisch durch eine Platte in den Fig.1 und 3 dargestellt. Demnach hat die Gehäusewand/der Haltebügel eine Durchgangsbohrung mit einem Bohrungsdurchmesser, der im Wesentlichen dem Außendurchmesser des Nutengrunds entspricht, wobei der Außendurchmesser des Nutengrunds vorzugsweise größer ist als der (durchschnittliche) Außendurchmesser des wenigstens einen Hülsenabschnitts 2, wie die insbesondere aus der Fig. 2 entnommen werden kann. Dabei kann der Nutengrund entweder von dem wenigstens einen Hülsenabschnitt 2 aus starrem Material oder von dem Kragen 12 aus flexiblem Material gebildet werden, wobei letzteres den Vorzug hat, Toleranzen des Bohrungsdurchmessers auszugleichen.

Mit Hilfe des Kragens/der Tülle 12 kann der erfindungsgemäße Schlauchverbinder 1 durch die Durchgangsbohrung/Ausbruch in der Gehäusewand/dem Haltebügel von einer Seite aus axial eingesteckt werden. Dabei greift die Geometrie des flexiblen Kragens 12 hinter die Wand/den Haltebügel, wodurch diese(r) in der Axialnut zwischen Kragen 12 und Axialanschlag 10 gehalten wird. Gleichzeitig werden die beiden einander zugewandten Dichtlippen 14, 16 gegen die Wand/den Haltebügel gedrückt, wodurch der Schlauchverbinder 1 zu der Wand/dem Haltebügel abgedichtet wird. Anschließend können die Schläuche wie in der Fig. 3 gezeigt auf sämtliche Hülsenabschnitte 2-6 aufgezogen werden.

Aufgrund der Flexibilität des Kragens 12 können Toleranzen in der Wandstärke gut ausgeglichen werden bzw. kann der Kragen 12 unterschiedliche Wandstärken abdecken. Zusätzlich kann der flexible Kragen 12 Vibrationen aufnehmen und Geräusche dämpfen. Schließlich können durch die Elastizität des Kragens 12 Bewegungen aufgrund von Temperaturschwankungen oder äußeren Erschütterungen abgefangen werden, ohne dass die Dichtigkeit zwischen sich und der Wand/dem Haltebügel verloren geht.

Abschließend sei darauf hingewiesen, dass der Ausbruch in der durchzuführenden bzw. zu befestigenden Wand/Haltebügel rotationssymmetrisch, nicht rotationssymmetrisch oder auch einfach als U-förmiger Ausbruch ausgeführt sein kann.

## Patentansprüche

1. Blutbehandlungsmaschine vorzugsweise der Dialysemaschinenbauart mit:
einer maschineninternen Verschlauchung zur fluidtechnischen Kopplung einer Anzahl von Maschinenelementen, welche an einem Maschinengehäuse und/oder an im Maschinengehäuse gelagerten Auszügen fixiert sind; und
mindestens einer Schlauchhalterung mit einem Schlauchverbindungselement (1) als fester Bestandteil der maschineninternen Verschlauchung, mittels welchem zwei Schläuche fluidtechnisch fest miteinander verbunden werden bzw. sind und/oder ein Schlauch in zwei oder mehrere weitere Schläuche verzweigt wird bzw. ist, wobei das Schlauchverbindungselement (1) zumindest zwei starre Hülsenabschnitte (2) umfasst, die zu einem einzigen starren Bauteil integral zusammengefasst sind und die dafür angepasst sind, um jeweils einen flexiblen Schlauch radial außenseitig über das freie Ende der zumindest zwei Hülsenabschnitte (2) aufzuziehen, wobei ein Axialanschlag (10) am Außenumfang wenigstens eines der Hülsenabschnitte (2) entfernt zu dessen freien Ende ausgebildet oder angeordnet ist, **gekennzeichnet durch** einen gegenüber dem starren Hülsenabschnitt (2) flexiblen Kragen (12), der im Axialabstand zum Axialanschlag (10) in Richtung hin zum freien Ende an dem Hülsenabschnitt (2) angeordnet oder ausgebildet ist, um zwischen sich und dem Axialanschlag (10) eine Außen-Umfangsnut zu definieren, wobei der Kragen (12) eine Dübelform hat, derart, dass der mit dem Kragen (12) versehene Hülsenabschnitt (2) des Schlauchverbindungselements (1) einfach von einer Seite in ein Aufnahmeloch einer Gehäusewand oder eines an der Gehäusewand montierten Haltebügels einsteckbar ist, und der Kragen (12) dann auf der anderen Seite selbsttätig und/oder federelastisch wieder aufspreizt und so die Gehäusewand oder den Haltebügel zwischen sich und dem Axialanschlag (10) axial hält.

2. Blutbehandlungsmaschine nach Anspruch 1, **dadurch gekennzeichnet, dass** sich die Dübelform des Kragens (12) in Richtung hin zum freien Ende des Hülsenabschnitts (2) verjüngt.

3. Blutbehandlungsmaschine nach einem der vorstehenden Ansprüche, **dadurch gekennzeichnet, dass** der Kragen (12) auf seiner dem Axialanschlag (10) zugewandten Stirnseite eine umlaufend geschlossene Dichtlippe (14) ausbildet oder hat, die axial in Richtung hin zum Axialanschlag (10) vorragt.

4. Blutbehandlungsmaschine nach Anspruch 3, **dadurch gekennzeichnet, dass** der Axialanschlag (10) auf seiner dem Kragen (12) zugewandten Stirnseite eine umlaufend geschlossene Dichtlippe (16) ausbildet oder hat, die axial in Richtung hin zum Kragen (12) vorragt.

5. Blutbehandlungsmaschine nach einem der vorstehenden Ansprüche, **dadurch gekennzeichnet, dass** der Kragen (12) aus einem Elastomer gefertigt ist, welches auf den Hülsenabschnitt (2) aufgespritzt, aufgeklebt oder aufgespannt ist.

6. Blutbehandlungsmaschine nach einem der vorstehenden Ansprüche, **dadurch gekennzeichnet, dass** der Nutengrund vom Kragen (12) gebildet wird.

7. Blutbehandlungsmaschine nach einem der vorstehenden Ansprüche, **dadurch gekennzeichnet, dass** das Schlauchverbindungselement (1) ein gerader Doppelstutzen, ein Winkelstück oder eine Y-, T- oder Kreuzweiche ist, bei welchen vorzugsweise nur einer der Mehrzahl von Hülsenabschnitten den Kragen (12) aufweist.

8. Blutbehandlungsmaschine nach einem der vorstehenden Ansprüche, **dadurch gekennzeichnet, dass** an wenigstens einer Gehäusewand, einem Auszug und/oder einem an einer Gehäusewand montierten Haltebügel ein Ausbruch in Form eines rotations-symmetrischen, rotations-unsymmetrischen und/oder U-förmigen Durchbruchs ausgebildet ist, dessen Durchmesser im Wesentlichen dem Nutengrund zwischen Kragen (12) und Axialanschlag (10) des Hülsenabschnitts (2) des Schlauchverbindungselements (1) der Schlauchhalterung entspricht.

9. Blutbehandlungsmaschine nach einem der vorstehenden Ansprüche, **dadurch gekennzeichnet, dass** die Dicke der wenigstens einen Gehäusewand, Auszugs und/oder des an einer Gehäusewand montierten Haltebügels im Wesentlichen der Nutenbreite zwischen Kragen (12) und Axialanschlag (10) des Hülsenabschnitts (2) des Schlauchverbindungselements (1) der Schlauchhalterung entspricht.

## Claims

1. A blood treatment machine, preferably of the dialysis machine type, comprising: a machine-internal hosing for the fluidic interconnection of a number of machine elements which are fixed to a machine housing and/or to racks supported in the machine housing; and
at least one hose mount comprising a hose connection element (1) as an inherent component of the machine-internal hosing, via which two hoses are fluidically firmly connected to each other and/or one hose is branched into two or more further hoses, wherein the hose connection element (1) comprises at least two rigid sleeve portions (2) integrally combined to form one rigid component and which are adapted to put a flexible hose radially outside over the free end of the at least two sleeve portions (2), wherein an axial stop (10) is formed or arranged on the outer circumference of at least one of the sleeve portions (2) at a distance from the free end thereof, **characterized by** a collar (12) which is flexible as compared to the rigid sleeve portion (2) and is arranged or formed on the sleeve portion (2) toward the free end with an axial distance from the axial stop (10) in order to define an external circumferential groove between itself and the axial stop (10), wherein the collar (12) has a dowel shape such that the sleeve portion (2) of the hose connection element (1) equipped with the collar (12) can be plugged from one side into a receiving hole of a housing wall or of a holding bracket mounted thereto, and the collar (12) automatically and in resilient fashion spreads on the other side and in this way axially holds the housing wall or the holding bracket between itself and the axial stop (10).

2. The blood treatment machine according to claim 1, **characterized in that** the dowel shape of the collar (12) tapers toward the free end of the sleeve portion (2).

3. The blood treatment machine according to one of the preceding claims, **characterized in that** the collar (12) forms or has a circumferentially closed sealing lip (14) on its front end facing the axial stop (10), said sealing lip protruding axially toward the axial stop (10).

4. The blood treatment machine according to claim 3, **characterized in that** the axial stop (10) forms or has a circumferentially closed sealing lip (16) on its front end facing the collar (12), said sealing lip protruding axially toward the collar (12).

5. The blood treatment machine according to one of the preceding claims, **characterized in that** the collar (12) is made from an elastomer material which is applied on the sleeve portion (2) by injection-molding, gluing or stretching.

6. The blood treatment machine according to one of the preceding claims, **characterized in that** the groove bottom is formed by the collar (12).

7. The blood treatment machine according to one of the preceding claims, **characterized in that** the hose connection element (1) is a straight double muff, an elbow piece or a Y-, T- or X-piece, wherein preferably only one of several sleeve portions (2) is provided with the collar (12).

8. The blood treatment machine according to one of the preceding claims, **characterized in that** a recess in the form of a rotationally symmetrical, rotationally asymmetrical and/or U-shaped opening is formed in at least one housing wall, a rack and/or a holding bracket mounted to a housing wall, the diameter of said recess substantially corresponding to the groove bottom between the collar (12) and the axial stop (10) of the sleeve portion (2) of the hose connection element (1) of the hose mount.

9. The blood treatment machine according to one of the preceding claims, **characterized in that** the thickness of the at least one housing wall, of the rack and/or of the holding bracket mounted to a housing wall substantially corresponds to the groove width between the collar (12) and the axial stop (10) of the sleeve portion (2) of the hose connection element (1) of the hose mount.

## Revendications

1. Machine de traitement du sang, de préférence de type machine de dialyse, avec : une tuyauterie interne à la machine pour le couplage fluidique d'un certain nombre d'éléments de machine, lesquels sont fixés à un boîtier de machine et/ou à des rallonges logées dans le boîtier de machine ; et au moins un support de tuyau avec un élément de connexion de tuyau (1) en tant que partie constituante fixe de la tuyauterie interne à la machine, au moyen duquel deux tuyaux vont être ou sont fermement connectés entre eux de manière fluidique et/ou un tuyau va être ou est ramifié en deux ou plusieurs tuyaux supplémentaires, dans laquelle l'élément de connexion de tuyau (1) comprend au moins deux sections de douille rigides (2) qui sont réunies d'un seul tenant en un seul composant rigide et qui sont adaptées pour enfiler respectivement un tuyau flexible radialement vers l'extérieur sur l'extrémité libre des au moins deux sections de douille (2), dans laquelle une butée axiale (10) est formée ou disposée sur la circonférence extérieure d'au moins une des sections de douille (2) à distance de son extrémité libre, **caractérisé par** un rebord flexible (12) en face de la section de douille rigide (2), qui est disposé ou formé sur la section de douille (2) à distance axiale de la butée axiale (10) en direction de l'extrémité libre pour définir une rainure circonférentielle extérieure entre elle et la butée axiale (10), dans laquelle le rebord (12) a une forme de cheville de sorte que la section de douille (2) de l'élément de connexion de tuyau (1) pourvu du rebord (12) peut être simplement insérée d'un côté dans un trou de réception d'une paroi de boîtier ou d'un étrier de maintien monté sur la paroi de boîtier, et le rebord (12) s'écarte ensuite de nouveau automatiquement de l'autre côté et/ou de manière élastique et maintient ainsi axialement la paroi de boîtier ou l'étrier de maintien entre elle et la butée axiale (10).

2. Machine de traitement du sang selon la revendication 1, **caractérisée en ce que** la forme de cheville du rebord (12) s'effile en direction de l'extrémité libre de la section de douille (2).

3. Machine de traitement du sang selon l'une quelconque des revendications précédentes, **caractérisée en ce que** le rebord (12) forme ou présente sur sa face frontale tournée vers la butée axiale (10) une lèvre d'étanchéité (14) fermée circonférentiellement, qui fait saillie axialement en direction de la butée axiale (10).

4. Machine de traitement du sang selon la revendication 3, **caractérisée en ce que** la butée axiale (10) forme ou présente sur sa face frontale tournée vers le rebord (12) une lèvre d'étanchéité (16) fermée circonférentiellement, qui fait saillie axialement en direction du rebord (12).

5. Machine de traitement du sang selon l'une quelconque des revendications précédentes, **caractérisée en ce que** le rebord (12) est constitué d'un élastomère, lequel est injecté, collé ou tendu sur la section de douille (2).

6. Machine de traitement du sang selon l'une quelconque des revendications précédentes, **caractérisée en ce que** le fond de rainure est formé par le rebord (12).

7. Machine de traitement du sang selon l'une quelconque des revendications précédentes, **caractérisée en ce que** l'élément de connexion de tuyau (1) est une double tubulure droite, un raccord coudé ou un aiguillage en Y, en T ou en croix, dans lesquels de préférence une seule de la pluralité de sections de douille présente le rebord (12).

8. Machine de traitement du sang selon l'une quelconque des revendications précédentes, **caractérisée en ce que** sur au moins une paroi de boîtier, une rallonge et/ou un étrier de maintien monté sur une paroi de boîtier, une coupe sous forme de perçage à symétrie de rotation, à asymétrie de rotation et/ou en forme de U est formée, dont le diamètre correspond sensiblement au fond de rainure entre le rebord (12) et la butée axiale (10) de la section de douille (2) de l'élément de connexion de tuyau (1) du support de tuyau.

9. Machine de traitement du sang selon l'une quelconque des revendications précédentes, **caractérisée en ce que** l'épaisseur d'au moins une paroi de boîtier, de la rallonge et/ou de l'étrier de maintien monté sur une paroi de boîtier correspond sensiblement à la largeur de rainure entre le rebord (12) et la butée axiale (10) de la section de douille (2) de l'élément de connexion de tuyau (1) du support de tuyau.
